**Europäisches Patentamt**

(19) **European Patent Office**

**Office européen des brevets**

(11) Veröffentlichungsnummer: **0 124 787**
**B1**

(12) **EUROPÄISCHE PATENTSCHRIFT**

(45) Veröffentlichungstag der Patentschrift:
12.08.87

(21) Anmeldenummer: 84103927.4

(22) Anmeldetag: 09.04.84

(51) Int. Cl.⁴: **A 23 J 3/00,** C 12 P 21/06,
C 12 N 1/00

(54) Stickstoffquelle für Organismen.

(30) Priorität: 13.04.83 DE 3313330

(43) Veröffentlichungstag der Anmeldung:
14.11.84 Patentblatt 84/46

(45) Bekanntmachung des Hinweises auf die Patenterteilung:
12.08.87 Patentblatt 87/33

(84) Benannte Vertragsstaaten:
AT BE CH DE FR GB IT LI LU NL SE

(56) Entgegenhaltungen:
EP-A-0 079 023
FR-A-1 568 432
GB-A-2 043 651

(73) Patentinhaber: HOECHST AKTIENGESELLSCHAFT,
Postfach 80 03 20, D-6230 Frankfurt am Main 80
(DE)

(72) Erfinder: Kachholz, Traudel, Dr., Schreyerstrasse
6, D-6242 Kronberg/Taunus (DE)
Erfinder: Scharf, Udo, Dr., Altenhainer Strasse 20,
D-6233 Kelkheim (Taunus) (DE)
Erfinder: Schlingmann, Merten, Dr., Schneidhainer
Strasse 32a, D-6240 Königstein/Taunus (DE)

## Beschreibung

Unter der Bezeichnung "Pepton" sind Proteinhydrolysate im Handel, die durch enzymatische Hydrolyse von Proteinen aus Fleisch, Milch oder Sojabohnen erhalten werden. Diese Peptone müssen eine Reihe von Anforderungen erfüllen: So darf im schwach sauren Bereich (pH 4) keine Trübung auftreten, beim Erhitzen in einem Autoklav (121°C bei 1 bar Überdruck) darf es keine Ausfällung geben, in stark bewegten Fermentern darf kein Schäumen hervorgerufen werden und das Mol-Gewicht muß in einem für Mikrobenwachstum günstigen Bereich liegen.

Bedingt durch die Proteinquelle schwankt die Zusammensetzung dieser Peptone innerhalb gewisser Grenzen, was nicht nur für wissenschaftliche, sondern insbesondere auch für technische Einsätze eine aufwendige Standardisierung erfordert. Es wurde nun gefunden, daß enzymatische Hydrolysate definierter mikrobieller Proteine diesen Nachteil nicht zeigen.

Die britische Patentanmeldung GB-A-2 043 651 beschreibt ein Verfahren zur Herstellung eines enzymatisch hergestellten, gereinigten Proteinhydrolysats, das in diverse Lebensmittel eingearbeitet werden kann. Die Verwendung des Hydrolysats als Stickstoffquelle für Organismen, wird jedoch nicht erwähnt. Das gleiche gilt für die Europäische Patentanmeldung EP-A-0 079 023.

Die Erfindung betrifft deshalb die Verwendung eines wasserlöslichen mikrobiellen Proteinhydrolysats, erhältlich durch enzymatische Hydrolyse der mikrobiellen Biomasse und Abtrennung der wasserlöslichen Fraktion mit einem Molekylargewicht bis zu 2000 Dalton, vorzugsweise von 400 bis 1500 Dalton als Pepton.

Diese Hydrolysate sind klar wasserlöslich, zeigen selbst bei pH 3 keine Trübung und erfüllen auch die vorstehend genannten Anforderungen hinsichtlich des Erhitzens im Autoklav und des Schäumens in stark bewegten Fermentern. Darüber hinaus hat es sich gezeigt, daß diese Hydrolysate im Vergleich zu Peptonen auf Fleisch- und Casein-Basis mindestens vergleichbare, in vielen Fällen sogar überlegene Stickstoffquellen darstellen. In jedem Falle haben die erfindungsgemäßen Hydrolysate eine einheitliche, reproduzierbare Zusammensetzung und damit einen definierten Stickstoff- und Aminosäure-Gehalt, da sie von einer einheitlichen Proteinquelle ausgehen.

Die Herstellung der erfindungsgemäßen Produkte erfolgt durch enzymatischen Abbau einheitlicher mikrobieller Zellmassen mit Hilfe von Endoproteasen und Isolierung der Eiweißfraktion im Mol-Gewichtsbereich bis zu 2000, vorzugsweise von 400 bis 1500, Dalton. Man kann die höhermolekularen Anteile - kontinuierlich oder absatzweise - in die enzymatische Hydrolyse zurückführen und so den Anteil der erwünschten niedermolekularen Fraktion entsprechend erhöhen.

Der enzymatische Abbau von Proteinen, auch solchen mikrobiellen Ursprungs, ist bekannt, ebenso die Auftrennung in Fraktionen nach dem Mol-Gewicht. Vorteilhafte Verfahrensbedingungen sind in der vorstehend genannten deutschen Offenlegungsschrift beschrieben.

Vorzugsweise wird eine wäßrige Suspension des mikrobiellen Proteins homogenisiert, auf den für das Enzym geeigneten pH-Wert eingestellt und unter Rühren auf die für das eingesetzte Enzym optimale Temperatur gebracht. Nach Zusatz des Enzyms wird die Suspension weiter gut durchmischt und so lange der Einwirkung des Enzyms ausgesetzt, bis der gewünschte Abbau erfolgt ist. Anschließend wird das Enzym in bekannter Weise inaktiviert, im allgemeinen durch Erhitzung, beispielsweise durch Einleiten vom Dampf.

Die Auftrennung des so erhaltenen Proteingemischs erfolgt nach üblichen Verfahren, vorteilhaft durch Membran-Trennverfahren, insbesondere Ultrafiltration. Membrantrennprozeße sind allgemein und insbesondere in der Biotechnologie geläufig (Übersichtsartikel: H. Strathmann, Chemie-Technik, 11 (1982), 813 - 819). Bevorzugt für die Herstellung der erfindungsgemäßen Produkte ist die Ultrafiltration in Platten-, Rohr-, Kapillarrohr-, Wickelmembran- und insbesondere Hohlfaserapparaten. Besonders geeignete Verfahren sind ebenfalls in der vorstehend genannten deutschen Offenlegungsschrift beschrieben.

Eine vorteilhafte Ausgestaltung der Erfindung besteht darin, daß man in einer ersten Stufe das mikrobielle Protein mit Trypsin abbaut, wobei ein Gewichtsverhältnis von Enzym : Substrat von etwa 1 : 500 eingehalten wird. Nach einer Abtrennung der höhermolekularen Fraktionen durch Hohlfaser-Ultrafiltration (Ausschlußgrenze 100 000 Dalton) wird des Permeat mit Alkalase behandelt, wobei ein Gewichtsverhältnis von Enzym : Substrat von etwa 1 : 50 eingehalten wird. Das Retentat (Konzentrat) wird hierbei anderweitig verwertet.

In einer anderen Ausführungsform der Erfindung werden die beiden genannten Enzyme im angegebenen Gewichtsverhältnis gleichzeitig eingesetzt und der Abbauprozeß einstufig oder zweistufig geführt.

Besonders zweckmäßig ist auch eine kontinuierliche Hydrolyse, wobei das Hydrolysat im Kreislauf über einen Ultrafiltrationsapparat geführt wird und das Retentat ständig in die Hydrolyse zurückgeführt wird.

Als Ausgangsmaterial kommen grundsätzlich alle mikrobiellen Zellmassen in Betracht, die technisch hergestellt werden und in einheitlicher Zusammensetzung anfallen. Bevorzugt sind methylotrophe, vor allem obligat methylotrophe Organismen, insbesondere Methanol verwertende Mikroorganismen, da Methanol eine billige, Erdöl-unabhängige Kohlenstoffquelle darstellt. Methanol verwertende Mikroorganismen sind in großer Zahl bekannt und beispielsweise in den europäischen Patentanmeldungen 35 831 und 37 273, in der deutschen Auslegeschrift 21 61 164 und insbesondere in der deutschen Patentschrift 26 33 451 beschrieben.

Mikroorganismen enthalten wie alle Zellen neben Proteinen und Kohlenhydraten auch Nukleinsäuren und Lipide. Bei der Aufarbeitung mikrobieller Zellmassen zur Gewinnung von Proteinen für die menschliche Ernährung werden außer den Nukleinsäuren auch die Lipide abgetrennt, da diese den Geruch, Geschmack und die Haltbarkeit der Proteine beeinträchtigen. Die Entfernung der Lipide erfolgt durch Extraktionen mit

# 0 124 787

Lipidlösemitteln. Entsprechende Verfahren sind beispielsweise in der deutschen Patentschrift 26 33 666 genannt. Besonders bevorzugt ist das in dieser deutschen Patentschrift beanspruchte Verfahren, bei dem die Lipide mit einer Extraktionsmischung aus Ammoniak und einem polaren Lösemittel aus der Reihe der niederen Alkanole, niederen Glycole oder der Methyl- oder Ethylether eines niederen Glykols extrahiert werden, wobei diese Extraktionsmischung höchstens 30 Gew.-% Wasser, bezogen auf die eingesetzte Lösemittelmenge, enthält. Das bevorzugte Lösemittel ist Methanol. Der Ammoniakgehalt liegt vorzugsweise bei 1 bis 10 Gew.-%, bezogen auf die eingesetzte Lösemittelmenge. Hierauf erfolgt eine ein- oder mehrstufige Extraktion der Nukleinsäuren mit Wasser.

Es ist vorteilhaft, wenn die mikrobiellen Zellmassen vor der Extraktion der Lipide durch eine Hitzebehandlung bei Temperaturen von 105 bis 160°C konditioniert werden. Diese Hitzebehandlung dauert vorteilhaft 3 bis 40, insbesondere 5 bis 20, Minuten bei einer Produkt-Temperatur von 105 bis 140°C. Ein solches Verfahren ist Gegenstand der Deutschen Patentanmeldung DE-A- 33 08 024; es führt nicht nur zu einer Verbesserung der mechanischen Eigenschaften der mikrobiellen Rohbiomasse, die einen geringeren Proteinverlust in der folgenden Extraktion ergibt, sondern hat auch einen vorteilhaften Einfluß auf die physiologischen Eigenschaften der Proteine.

Für die folgenden Beispiele wurde eine Bakterienmasse aus Methylomonas clara ATCC 31 226 gemäß Deutscher Patentschrift 26 33 451 (U.S. Patentschrift 4 166 004), Beispiel 2, mit einem Nukleinsäuregehalt von 8 - 10 %, einem Rohfettgehalt von 5 bis 10 % und einer Restfeuchte von 2 bis 4 % eingesetzt. Dieses Material wurde 30 Minuten in einem Wirbelbett bei 160°C Lufttemperatur behandelt, wobei 10 Minuten eine Produkttemperatur von 120°C eingehalten wurde. Diese thermisch nachbehandelte Zellmasse wurde dann gemäß Beispiel 1 der deutschen Patentschrift 26 33 666 (U.S. Patentschrift 4 206 243) mit methanolischem Ammoniak und anschließend mit Wasser extrahiert. Prozentangaben beziehen sich hier und im folgenden auf das Gewicht.

## Beispiel 1

Ein einmal mit Wasser extrahiertes Ausgangsmaterial mit einem Restnukleinsäuregehalt von 3 bis 5 % wird auf 5 % Feststoffgehalt verdünnt, mit verdünnter Natronlauge auf pH 8,0 eingestellt und die Mischung auf 50°C erhitzt. Pro Liter Suspension werden 0,1 g Trypsin PTN 3.0 S und 1 g Alkalase 0.6 L zugegeben und die Mischung 2 Stunden lang gerührt, wobei Temperatur und pH-Wert - letzterer durch Zugabe verdünnter Natronlauge - konstant gehalten werden. Hierauf werden nochmals die gleichen Mengen der genannten Enzyme zugesetzt und pH-Wert und Temperatur weitere 2 Stunden unter Rühren aufrecht erhalten.

Durch Erhitzen auf 80°C werden die Enzyme inaktiviert und das Hydrolysat im Kreislauf über einen Hohlfaser-Ultrafiltrationsapparat (Firma Romicon PM 100) bei einem Durchfluß von 6000 l pro Stunde bei 1,8 bar und einem Permeatabfluß von 500 bis 250 l pro Stunde fraktioniert. Das die Membran nicht passierende Retentat wird in die Vorlage zurückgeführt und das abfließende Permeat über einen Fallstromverdampfer auf 20 % Trockensubstanz aufkonzentriert und sprühgetrocknet. Man erhält etwa 60 % Pepton, das in Wasser klar löslich ist und bei pH 3 keine Trübung zeigt.

## Beispiel 2

Ein zweimal mit Wasser extrahiertes Ausgangsmaterial mit einem Restnukleinsäuregehalt von 2 % wird auf 10 % Feststoffgehalt verdünnt, mit verdünnter Natronlauge auf pH 8,0 eingestellt und die Suspension auf 50°C erhitzt. Hierauf werden 0,2 g Trypsin PTN 3,0 S pro Liter zugesetzt und die Mischung 4 Stunden gerührt, wobei die Temperatur und der pH-Wert (durch Zugabe verdünnter Natronlauge) konstant gehalten werden. Zur Inaktivierung des Enzyms wird auf 80°C erhitzt und das Hydrolysat im Kreislauf über den im Beispiel 1 genannten Hohlfaser-Ultrafiltrationsapparat geführt. Das nach mehrmaligem Durchfluß die Membran nicht passierende Retentat wird sprühgetrocknet und als Lebensmittelzusatz verwendet.

Das Permeat wird mit 1/50 des Feststoffgewichts an Alkalase 0.6 L versetzt und nochmals 4 Stunden bei 50°C inkubiert. Hierauf wird durch Erhitzen auf 80°C die Alkalase inaktiviert. Das Hydrolysat wird über einen Fallstromverdampfer auf 20 % Trockensubstanz aufkonzentriert und sprühgetrocknet. Man erhält etwa 30 % des eingesetzten Feststoffgewichts an Pepton.

Die folgende Tabelle 1 zeigt weitere Beispiele für die Durchführung der Hydrolyse. Gearbeitet wurde nach Beispiel 2 mit den in der Tabelle genannten Variationen.

**Tabelle 1**

| Beisp. | Substrat-Konz., % | Enzym | Verh.* | % Ausb. Permeat |
|---|---|---|---|---|
| 3 | 2 | Trypsin PTN 3.OS | 100 | |
| | | Alkalase 0.6 L | 10 | 77,1 |
| 4 | 10 | Trypsin PTN 3.OS | 500 | |
| | | Pronase E | 1000 | 55,7 |
| 5 | 5 | Trypsin PTN 3.OS | 50 | |
| | | Alkalase 0.6 L | 5 | 72,2 |

* Gewichtsverhältnis Substrat/Enzym.

Das nach Beispiel 1 erhaltene Pepton wurde - im Vergleich mit Casein- und Fleischpepton - in Nährböden der folgenden Zusammensetzung eingearbeitet:

**Tabelle 2**
Nährböden für (in g/l dest. Wasser)

Milchsäurebakterien
Brevibacterium
Corynebacterium

| Propionibacterium | | Sonst. Bakterien | | Hefen und Schimmelpilze | |
|---|---|---|---|---|---|
| Fleischextrakt | 5,0 | Fleischextrakt | 5,0 | Malzextrakt | 30,0 |
| Hefeextrakt | 5,0 | $Na_2HPO_4$ | 2,0 | Pepton | 3,0 |
| Glucose | 20,0 | NaCl | 3,0 | | |
| $K_2HPO_4$ | 2,0 | Pepton | 10,0 | | |
| $(NH_4)_2$ H-Citrat | 2,0 | | | | |
| $CH_3COONa$ | 5,0 | | | | |
| $MgSO_4$ | 0,1 | | | | |
| $MnSO_4$ | 0,05 | | | | |
| Pepton | 10,0 | | | | |
| pH | 7,0 | pH | 7,0 | pH | 5,5 |

Auf diesen Nährböden wurden die in der folgenden Tabelle 3 genannten Mikroorganismen gezüchtet:

Tabelle 3 a: Wachstum auf: (O.D./30 ml)

| Organismus | erfindungsgemäßem Pepton | | | | | Fleischpepton | | | | | Caseinpepton | | | | |
|---|---|---|---|---|---|---|---|---|---|---|---|---|---|---|---|
| | 1 d | 2 d | 3 d | 4 d | 5 d | 1 d | 2 d | 3 d | 4 d | 5 d | 1 d | 2 d | 3 d | 4 d | 5 d |
| Rhodococcus erythropolis | 14,5 | 14,5 | 17,0 | 16,5 | 15,5 | 10,0 | 13,5 | 13,0 | 13,5 | 12,0 | 8,5 | 13,0 | 12,0 | 13,5 | 17,0 |
| Micrococcus caseolyticus | 1,0 | 0,5 | 1,5 | 1,0 | 0,8 | 0,5 | 1,0 | 3,0 | 2,0 | 0,7 | 2,0 | 2,5 | 2,5 | 2,0 | 1,5 |
| Bacillus licheniformis | 6,0 | 10,0 | 12,5 | 11,0 | 9,35 | 9,5 | 7,5 | 11,5 | 10,5 | 8,4 | 9,0 | 6,5 | 6,0 | 7,5 | 6,5 |
| Escherichia coli | – | 2,02 | 1,92 | 1,97 | 2,25 | – | 1,82 | 1,53 | 1,78 | 2,57 | – | 1,29 | 1,32 | 1,20 | 1,21 |
| Klebsiella pneumoniae | – | 3,58 | 4,04 | 3,97 | 3,93 | – | 1,40 | 1,15 | 1,24 | 1,76 | – | 2,79 | 2,83 | 2,87 | 1,24 |
| Pseudomonas aeruginosa | – | 1,82 | 1,97 | 1,95 | 2,56 | – | 2,44 | 1,89 | 2,59 | 3,9 | – | 1,91 | 2,08 | 2,08 | 3,27 |
| Lactobacillus buchneri | 5,0 | 8,6 | 5,4 | 5,7 | 6,3 | 7,4 | 13,8 | 6,6 | 5,9 | 5,1 | 6,8 | 7,8 | 6,5 | 15,9 | 5,6 |
| Brevibacterium linens | 5,2 | 7,2 | 8,4 | 8,0 | 8,4 | 0,6 | 6,0 | 6,8 | 7,1 | 6,8 | 0,8 | 7,6 | 7,4 | 6,9 | 6,2 |
| Leuconostoc cremoris | 5,4 | 5,8 | 6,2 | 5,4 | 5,4 | 5,2 | 5,8 | 5,4 | 5,0 | 4,9 | 4,6 | 5,2 | 4,6 | 4,6 | 4,7 |

0124787

Tabelle 3 b:  Wachstum auf:  (O.D./30 ml)

| Organismus | erfindungsgemäßem Pepton | | | | | Fleischpepton | | | | | Caseinpepton | | | | |
|---|---|---|---|---|---|---|---|---|---|---|---|---|---|---|---|
| | 1 d | 2 d | 3 d | 4 d | 5 d | 1 d | 2 d | 3 d | 4 d | 5 d | 1 d | 2 d | 3 d | 4 d | 5 d |
| Debaryomyces hansenii | 2,8 | 19,6 | 28,5 | 37,5 | 40,0 | 2,5 | 11,0 | 24,0 | 25,0 | 28,0 | 2,2 | 10,4 | 11,0 | 15,0 | 14,0 |
| Kluyveromyces fragilis | 6,9 | 3,4 | 10,0 | 10,0 | 11,0 | 3,9 | 3,8 | 10,0 | 11,0 | 12,0 | 3,7 | 6,8 | 13,5 | – | 11,0 |
| Candida rugosa | 6,8 | 6,6 | 8,0 | 8,0 | 7,0 | 7,0 | 5,4 | 10,5 | 9,0 | 7,5 | 6,4 | 7,0 | 8,5 | 8,0 | 9,0 |
| Hansenula subpelliculosa | 12,5 | 35,6 | 46,5 | 60,0 | 67,0 | 12,3 | 29,6 | 47,0 | 64,0 | 60,0 | 16,5 | 33,2 | 48,5 | 52,0 | 53,0 |
| Saccharomyces cerevisiae | 6,4 | 10,6 | 25,0 | 24,0 | 25,0 | 9,2 | 11,0 | 24,5 | 26,0 | 26,0 | 9,5 | 20,4 | 22,5 | 28,0 | 34,0 |

Tabelle 3 c:  Wachstum auf:  (Trockengewicht/100 ml)

| Organismus | erfindungsgemäßem Pepton | | | | Fleischpepton | | | | Caseinpepton | | | |
|---|---|---|---|---|---|---|---|---|---|---|---|---|
| | 2 d | 4 d | 6 d | 8 d | 2 d | 4 d | 6 d | 8 d | 2 d | 4 d | 6 d | 8 d |
| Aspergillus tamarii | 0,78 | 0,79 | 0,82 | 0,50 | 0,51 | 0,074 | 0,83 | 0,84 | 1,05 | 0,92 | 0,80 | 0,70 |
| Penicillium cambertii | 0,03 | 0,03 | 0,06 | 0,10 | 0,03 | 0,02 | 0,05 | 0,08 | 0,03 | 0,04 | 0,05 | 0,07 |
| Rhizopus oryzae | 0,37 | 0,48 | 0,53 | 0,51 | 0,40 | 0,32 | 0,57 | 0,59 | 0,45 | 0,62 | 0,57 | 0,57 |
| Stereum hirsutum | 0,03 | 0,05 | 0,17 | 0,74 | 0,05 | 0,07 | 0,15 | 0,19 | 0,04 | 0,07 | 0,33 | 0,47 |

0124787

**0 124 787**

**Patentansprüche**

Verwendung eines wasserlöslichen mikrobiellen Proteinhydrolysats, erhältlich durch enzymatische Hydrolyse der mikrobiellen Biomasse und Abtrennung der wasserlöslichen Fraktion mit einem Molekulargewicht bis zu 2000 Dalton, als Pepton.

**Claims**

The use of a water soluble microbial protein hydrolyzate, obtainable by enzymatic hydrolysis of microbial biomass and separation of the water soluble fraction with a molecular weight of up to 2000 dalton, as a peptone.

**Revendications**

Utilisation d'un hydrolysat de protéines microbiennes soluble dans l'eau, préparable par hydrolyse enzymatique de la biomasse microbienne et séparation de la fraction soluble dans l'eau, ayant un poids moléculaire allant jusqu'à 2000 daltons, en tant que peptone.

7